**Europäisches Patentamt**

**European Patent Office**

**Office européen des brevets**

(19)

(11) Publication number: **0 235 817**
**A2**

(12)

# EUROPEAN PATENT APPLICATION

(21) Application number: **87103140.7**

(22) Date of filing: **05.03.87**

(51) Int. Cl.⁴: **C 12 P 21/00, G 01 N 33/577**

(30) Priority: **06.03.86 JP 48800/86**

(43) Date of publication of application: **09.09.87**
**Bulletin 87/37**

(84) Designated Contracting States: **DE FR GB**

(71) Applicant: **KYOWA HAKKO KOGYO CO., LTD., 6-1, Ohte-Machi 1-chome, Chiyoda-ku Tokyo-to (JP)**

(72) Inventor: **Yoshida, Hajime, 9-18, Ishobe, Sagamihara-shi Kanagawa (JP)**
Inventor: **Hanai, Nobuo, 9056 Easthorewooddrive, Apt. No. 246, Mercer Island Washington 98040 (US)**
Inventor: **Kumagai, Akiko, 7-4-2, Tamagawagakuen, Machida-shi Tokyo (JP)**

(74) Representative: **Kinzebach, Werner, Dr., Patentanwälte Reitstötter, Kinzebach & Partner Sternwartstrasse 4 Postfach 86 06 49, D-8000 München 86 (DE)**

(54) **Anti-human stomach cancer monoclonal antibody.**

(57) An anti-human stomach cancer monoclonal antibody, AMC-382, which belongs to the class IgM and is capable of reacting with human stomach cancer cells, non-reactive with normal human stomach tissues (or cells) and capable of recognizing the sugar chains of sialylated glycoproteins or glycolipids as antigens is disclosed. It is useful in sero-diagnosis of human stomach cancer.

EP 0 235 817 A2

ANTI-HUMAN STOMACH CANCER MONOCLONAL ANTIBODY

## BACKGROUND OF THE INVENTION

### Field of the Invention

This invention relates to a novel anti-human stomach cancer monoclonal antibody, AMC-382, and to a method of serodiagnosis of stomach cancer using the same.

### Description of the Prior Art

Methods of producing monoclonal antibodies to human stomach cancer are known which comprise propagating hybridomas derived from antibody-producing cells by fusion with myeloma cells [Taisha (Metabolism and Disease), 21 Special Supplement, 819-824 (1984); Cancer Research, 44, 3952-3956 (1984); Gann (Japanese Journal of Cancer Research), 75, 106-108 (1984); Japanese Patent Application Laid-Open No. 59-128397].

There is also a report on serodiagnosis using anti-human stomach cancer monoclonal antibodies [Proceedings of the 44th General Meeting of the Japanese Cancer Society, page 131 (1985)].

As serodiagnostic kits for spleen cancer which use monoclonal antibodies capable of recognizing sialylated sugar chains (oligosaccharide side chains), there are known NS19-9 [International Journal of Cancer, 33, 339 (1984); Rinsho Byori (Japanese Journal of Clinical Pathol-

ology), XXXI, <u>12</u>, 1300 (1983)] and DuPan-2 [Proc. Natl.

Acad. Sci. USA, <u>81</u>, 5242 (1984); Kan-Tan-Sui (Liver, Gall

Bladder and Spleen), <u>11</u>, 37 (1985)].

At present the percent detection of early stomach cancer (at stage I and stage II) in Japan is reportedly about 35% [Gan to Kagaku Ryoho (Cancer and Chemotherapy),

<u>11</u> (3), Part II, 716-726 (1984)].

No diagnostic means have been developed as yet for simple and rapid detection of early stomach cancer.

As a result of their studies in search of monoclonal antibodies specifically reactive with stomach cancer, the present inventors succeeded in producing a monoclonal antibody which shows particularly strong reactivity with stomach cancer and is applicable in serodiagnosis of stomach cancer with relatively high positive rates even in the case of early stomach cancer, thus the present invention has now been completed.

## SUMMARY OF THE INVENTION

The present invention provides an anti-human stomach cancer monoclonal antibody, AMC-382, which belongs to the class IgM and is capable of reacting with human stomach cancer cells, non-reactive with normal human stomach tissues (or cells) and capable of recognizing the sugar chains of sialylated glycoproteins or glyco-

lipids as antigens.

The monoclonal antibody according to the present invention can be produced by preparing a hybridoma resulting from fusion of mouse myeloma cells with spleen cells of a mouse immunized with human stomach cancer cells or tissues or membrane components and cultivating the hybridoma in a medium or administering the hybridoma to a mouse to thereby cause hybridoma cell propagation in the mouse's ascitic fluid, followed by separation of the antibody from the culture or ascitic fluid.

The invention also includes serodiagnostic methods for detecting the presence of human stomach cancer by analyzing a serum sample from the patient as well as an immunohistochemical staining procedure for selectively staining a tissue sample from the stomach of a patient suspected of having stomach cancer. Both procedures are highly selective in that the AMC-382 monoclonal antibody does not react with normal human stomach tissues or cells. Serodiagnosis test kit compositions are also described.

<u>BRIEF DESCRIPTION OF THE DRAWINGS</u>

In the accompanying drawings,

Fig. 1 is a scatter chart showing the results of serodiagnosis of stomach cancer using the monoclonal antibody AMC-382;

Fig. 2 is a scatter chart showing the influence of the stage of stomach cancer upon the results of serodiagnosis of stomach cancer with the monoclonal antibody AMC-382; and

Fig. 3 is a graph showing the changes in reactivity of stomach cancer tissue-derived membrane components with the monoclonal antibody AMC-382 upon treatment with various enzymes and a reagent. The symbols used are as follows: ● for treatment with 0.1 U/ml of neuraminidase, Δ for treatment with 0.1 U/ml of α-L-fucosidase, □ for treatment with 0.25% trypsin, ◼ for treatment with 10 U/ml of protease, o for treatment with 50 mM $NaIO_4$, and x for no treatment.

## DETAILED DESCRIPTION OF THE INVENTION

In the following, a method of producing the monoclonal antibody according to the invention will be described in detail.

(1)  Immunization of animal and preparation of antibody-producing cells

Mice  3-10  weeks of age, preferably 8 weeks of age, are immunized with human stomach cancer cells, tissues or membrane components to thereby prepare antibody-producing cells in the spleen, lymph node and peripheral blood of the animals. Mice rendered immunologically tolerant by pretreatment with normal human stomach cells should preferably be used as the mice to be immunized. The immunization is performed by administering human stomach cancer

- 4 -

cells ($10^6$ to $10^7$ cells per animal), human stomach cancer tissues or membrane components (membrane fragments) derived therefrom (10-500 µg per animal) together with an appropriate adjuvant (e.g. Freund's complete adjuvant, or aluminum hydroxide gel plus pertussis vaccine) subcutaneously, intraveously or intraperitoneally. Thereafter, antigen administration is repeated 2-5 times at one- to two-week intervals. Three to seven days after each immunization, the blood is sampled from the eyeground venous plexus and the serum of each sample is tested as to whether it reacts with human stomach cancer by the enzyme immunoassay technique described below [Enzyme-linked Immunosorbent Assay (ELISA), published by Igaku Shoin, Tokyo, 1976], for instance.

Normal human and tumor tissues were obtained from autopsies or surgical operations. The tissues were immediately frozen and stored at -80°C. For membrane components the tissues were thawed at 4°C in PBS containing 1 mM phenylmethyl sulfonyl fluoride. After mincing they were disrupted with an ultra disperser (LK-21; Yamato, Tokyo, Japan) and homogenized with a teflon-glass homogenizer. The homogenate was centrifuged at 100,000 x g, and then the pellet was resuspended at 1 mg protein in 1 ml of PBS and stored at -80°C.

Enzyme immunoassay technique:

The membrane components of normal or tumor cells or tissues (membrane fragment fraction containing 10-1,000 µg of proteins per milliliter) are distributed into wells of a 96-well plate for EIA (Flow Laboratories) in an amount of 100-200 µl per well. After allowing to stand for overnight to two-overnights at 4°C, the plate well bottom is coated with bovine serum albumin. Each well is deprived of the supernatant and then washed well with deionized water or phosphate-buffered saline (PBS; 1.83 g of disodium phosphate, 0.21 g of monopotassium phosphate and 7.65 g of sodium chloride in each liter of distilled water, pH 7.2). Then, 1% BSA (bovine serum albumin)-PBS is distributed, as the first antibody, into the wells in an amount of 100-200 µl per well and the residual protein-binding sites on the plate are bloched by allowing to stand at 4°C for overnight to two-overnights  Thereafter, the BSA-PBS is discarded and the wells are washed well with deionized water or PBS. Samples (mouse sera, hybridoma culture supernatants, or roughly purified monoclonal antibodies, each as the first antibody) are diluted with BSA-PBS and the dilutions are distributed into the wells in an amount of 100 µl per well, followed by overnight standing at 4°C. After washing the wells with one portion of deionized water and then with six portions of 2 M NaCl solution, a 100-fold dilution of

- 6 -

the rabbit anti-mouse immunoglobulin IgG-peroxidase conjugate (produced by DAKO and distributed by Kyowa Medex) is distributed, as the second antibody, into the wells in an amount of 100 μl per well. The plate is then allowed to stand at room temperature for 2 hours.

After washing well with PBS, an ABTS substrate solution [prepared by dissolving 550 mg of 2,2'-azinobis-(3-ethylbenzothiazoline-6-sulfonic acid) diammonium salt in 1 liter of 0.1 M citrate buffer (pH 4.2) and adding, just prior to use, hydrogen peroxide to a concentration of 1 μl/ml] is applied and the color developed is measured in terms of the absorbance $OD_{415nm}$. Those mice that react strongly with the stomach cancer cells, tissues or membrane components are used as human stomach cancer-immunized mice, namely as sources of supply of antibody-producing cells for the hybridoma production.

When cells as they are are used as the antigen in performing the enzyme immunoassay, the target cells are cultivated on a Falcon 3072 plate, 0.25% glutaraldehyde-PBS is added and, after allowing it to stand at room temperature for 1-2 hours, the plate is washed well with PBS. Then, 100-200 μl of 1% BSA-PBS is added and, after 2 hours of standing, the plate is washed well with deionized water or PBS and submitted to antibody titer determination, which is conducted in the same manner as the case where an ordinary antigen-coated plate is used.

For submitting to cell fusion, spleen cells are prepared by intraperitoneally administering human stomach cancer cells, tissues or membrane components in a dose of 2 to 5 x 10$^6$ cells per animal or 20-400 μg per animal to the immunized mice 3-4 days prior to fusion treatment and thereafter excising the spleen. Thus, the spleen is cut to pieces in MEM (minimal essential medium; Nissui Pharmaceutical), loosened up with a pair of forceps and centrifuged at 1,200 rpm for 5 minutes. The supernatant is discarded, and the cells are deprived of erythrocytes by treatment with Tris-ammonium chloride buffer (pH 7.65) for 1-2 minutes, washed with three portions of MEM and used as the spleen cells for fusion.

(2) Preparation of myeloma cells

A mouse-derived established myeloma cell line is used. Usable examples of such cell line are the 8-azaguanine resistant mouse (BALB/c-derived) myeloma cell lines P3-X63Ag8-U1 (P3-U1) [Current Topics in Microbiology and Immunology - 1] [European Journal of Immunology, 6, 511-519 (1976)], SP2/0-Ag14 (SP-2) [Nature, 276, 269-270 (1978)], P3-X63-Ag8653 (653) [Journal of Immunology, 123, 1548-1550 (1979)] and P3-X63-Ag8 (X-63) [Nature, 256, 495-497 (1975)]. The passage of these cell lines is performed in 8-azaguanine medium [normal medium prepared by adding, to RPMI-1640 medium, glutamine (1.5 mM), 2-mercaptoethanol

$(5 \times 10^{-5}$ M), gentamicin (10 µg/ml) and fetal calf serum (FCS; product of CSL) (10%), with further supplementation with 8-azaguanine (15 µg/ml)]. The cell line selected for cell fusion should be transferred to normal medium 3-4 days before fusion to ensure a cell count of not less than $2 \times 10^{7}$ on the day of fusion.

(3) Cell fusion

The antibody-producing cells immunized above in (1) and the myeloma cells obtained above in (2) are washed well with MEM or PBS and mixed in a cell number ratio of antibody-producing cells : myeloma cells = 5 to 10 : 1 and then centrifuged (1,200 rpm, 5 minutes). The supernatant is discarded and the cell sediment is loosened up. A mixture of 2 g of polyethylene glycol 1000 (PEG-1000), 2 ml of MEM and 0.7 ml of dimethyl sulfoxide is added, in an amount of 0.2-1 ml per $10^{3}$ antibody-producing cells, to the mixed cells at 37°C with stirring. The whole volume is made 50 ml by several additions of 1-2 ml of MEM at one- to two-minute intervals. After centrifugation (900 rpm, 5 minutes), the supernatant is discarded and the cell sediment is loosened gently. To the cells is added 100 ml of normal medium (RPMI 1640 with 10% FCS). The cells are suspended in the medium by gently drawing up into and discharging from a measuring pipette.

The suspension obtained is distributed, in 1-ml portions, into the wells of a 24-well incubation plate. Incubation is carried out in a 5% $CO_2$ incubator at 37°C for 24 hours. HAT medium [normal medium supplemented with hypoxanthine ($10^{-4}$ M), thymidine ($1.5 \times 10^{-5}$ M) and aminopterine ($4 \times 10^{-7}$ M)] is added to the incubation plate (1 ml per well) and incubation is conducted for an additional 24 hours. Thereafter, 1 ml of the culture supernatant is discarded and the same volume of fresh HAT medium is added instead at 24-hour intervals for 2 days. The incubation in the $CO_2$ incubator is continued at 37°C for 10-14 days.

For those wells in which fused cells grown and forming colonies are found, 1 ml of the supernatant is discarded and the same volume of HT medium (HAT medium minus aminopterine) is added, followed by medium replacement with a fresh portion of HT medium at 24-hour intervals for 2 days.

After 3-4 days of cultivation in HT medium, a portion of the culture supernatant is collected and assayed for antibody titer relative to human stomach cancer by the above-mentioned enzyme immunoassay technique. Simultaneously, the reactivities with normal human cells or tissues or membrane components, among others, are also determined by the same method, and those wells for which selective reactivity with human stomach cancer cells, tissues or membrane components is found are selected. For those wells which show strong reactivity with human stomach cancer cells, tissues or membrane components but no reactivity with normal human cells, tissues or membrane components, among others, cloning is repeated twice by the limiting dilution technique. In this manner, those clones with which high antibody titer values are constantly obtained relative to human stomach cancer cells or tissues or membrane components are selected as anti-human stomach cancer monoclonal antibody-producing hybridoma cell lines.

(4) Preparation of monoclonal antibodies

Eight- to ten-week-old, female C57BL/6 mice treated with pristane [intraperitoneally administered with 0.5 ml of 2,6,10,14-tetramethylpentadecane (pristane) and fed for 2 weeks] are intraperitoneally injected with the anti-human stomach cancer monoclonal antibody-producing hybridoma cells obtained above in (3) in a dose of 2-4 x $10^6$ cells per animal. In 10-21 days, the hybridoma cells produce ascites carcinoma in the mice. The ascitic fluid is collected from such a mouse, centrifuged (3,000 rpm, 5 minutes) to remove solids, subjected to salting out with 50% ammonium sulfate, dialyzed against PBS with NaCl added to 0.5 M, and applied to a Sephacryl S300 (Pharmacia Fine Chemicals) column (bed volume 750 ml) at a flow rate of 15 ml/hr. An IgM fraction is collected and used as a purified monoclonal antibody fraction.

The isotype of the antibody is determined by Ouchterlony's method (double immunodiffusion) [Seikagaku Jikkenho (Methods in Experimental Biochemistry), vol. 15, Introduction to Experimental Immunology, p. 74, Gakkai Shuppan Center, 1981].

The protein content is determined by the Folin method, followed by calculation based on the absorbance at 280 nm [1.4 ($OD_{280}$) is approximately equal to 1 mg of immunoglobulin per milliliter].

The monoclonal antibodies thus obtained are evaluated for specficity characteristics based on the reactivities with normal and tumor tissues and membrane components thereof as derived from a variety of human organs obtained from a plurality of subjects, the reactivities with a variety of cultured human normal or tumor cell lines and membrane components derived therefrom, the reactivities with hitherto known carcinogenic embryonic antigens (e.g. CEA) and the reactivities with normal human-derived and patient-derived sera, among others, as determined by the immunoassay method, fluorescent antibody method, immunohistological evaluation method (PAP method), etc., and those monoclonal antibodies that will not show any substantial reactivity except for human stomach cancer in any evaluation test are selected.

The thus-obtained monoclonal antibodies which react specifically with human stomach cancer are expected to be useful in diagnosis of stomach cancer by serologic examination, histologic examination, imaging, etc., or in the treatment of stomach cancer which comprises administering the antibodies as they are or in the form of the so-called immunotoxins, namely conjugates with anticancer agents or toxins, to cancer patients. Furthermore, it is expected that these tumor-specific monoclonal antibodies might contribute, through the use of affinity columns in which they are used, to the purification of tumor-specific antigens, to the analysis of such antigens and further to the development of stomach cancer vaccine.

(5)  Serodiagnosis of stomach cancer

The serodiagnosis may be performed in the following manner:

Enzyme immunoassay by the sandwich method (ELISA)

A first antibody preparation (10-100 µg/ml) is distributed into the wells of a 96-well plate for EIA (50-200 µl per well). The plate is allowed to stand at 4°C for overnight to two-overnights  or at room temperature for 2-4 hours. After washing with PBS, 200 µl of BSA-PBS is added to each well, followed by further standing at 4°C overnight or at room temperature for 2 hours. The plate is then washed well with PBS, and 50-100 µl of a 1- to 100-fold dilution of a serum sample is added to each well. After allowing to stand at 4°C overnight or at room temperature for 2 hours, the plate is then washed well with PBS. Then, a biotin- or peroxidase-labeled second antibody (10-100 µg/ml) is added to the wells (50-100 µl per well) and the plate is further allowed to stand at 4°C overnight or at room temperature for 2-4 hours. When a biotin-labeled antibody is used as the second antibody, the plate is washed well with PBS, avidin-biotin-peroxidase (10 µg/ml) is added to the wells (50-100 µl per well), and the plate is allowed to stand at room temperature for 30 minutes and then washed well with PBS. Then, an ABTS substrate solution is added, as the substrate solution, in an amount of 50-100 µl per well. After allowing to stand at room temperature for 10-30 minutes, the reaction is terminated by adding 5% SDS (sodium dodecyl sulfate) solution in an amount of 50-100 µl per well. The $OD_{415}$ value is measured for each well and the antigen quantity in the serum sample is calculated based on the intensity of the color developed. By comparing the antigen levels in the sera of healthy subjects with those in the sera of patients with stomach cancer, a normal level range is defined. When the level in question exceeds this range, the test for stomach cancer is regarded as positive.

Radioimmunoassay (RIA)

An AMC-382 preparation (10-100 µg/ml) is distributed, as the first antigen, into the U-shaped bottom wells of a 96-well disposable plate (50-200 µg per well), and the plate is allowed to stand at 4°C for overnight to two-overnights or at room temperature for 2-4 hours. After washing with PBS, BSA-PBS is added to the wells in an amount of 200 µl per well, followed by further standing at 4°C overnight or at room temperature for 2 hours. The plate is washed well with PBS, and a 1- to 100-fold dilution of a serum sample is added to each well in an amount of 50-100 µl. After allowing to stand at 4°C overnight or at room temperature for 2 hours, the plate is washed well with PBS.

Then, an $^{125}$I-labeled second antibody ($^{125}$I-AMC-382) is added to the wells in an amount of 50-100 µl per well. After further standing at 4°C overnight or at room temperature for 2-4 hours, the plate is washed well and then air-dried. Each well is cut off using a hot Nichrome wire, placed in a Shionogi tube and measured for radioactivity using a gamma-counter (Aroka), and the antigen quantity in each serum sample is calculated based on the cpm (counts per minute) value obtained.

While serodiagnosis of stomach cancer can be performed in the above manner, the serodiagnosis of stomach cancer using the monoclonal antibody AMC-382 is by no means limited to the modes mentioned above.

(6)    Antigen analysis

When, in performing the above-mentioned enzyme immunoassay, immunohistochemical staining or serodiagnosis, the
antigens (stomach cancer membrane components, cultured
stomach cancer cell lines, stomach cancer tissues) are
pretreated with reagents such as neuraminidase, protease
or other enzymes or periodic acid and then reacted with
the monoclonal antibody, the subsequent comparison for
differences in reactivity with the monolconal antibody
between the original antigens without such pretreatment
and the antigens pretreated in the above manner can throw
light on the chemical characteristics of the antigens, that
is the chemical characteristics of the antigenic sites which the
monoclonal antibody can recognize.    Thus, if the anti-
genicity disappears upon treatment with neuraminidase,
it is estimated that sialic acids are associated with the
antigenic determinants.    If the antigenicity disappears
upon treatment with protease, proteins are supposed to be
associated with the antigenic determinants.    If the antigenicity disappears upon treatment with periodic acid,
sugar chains may be associated with the antigenic determinants.

The following non-limiting examples illustrate the invention in
further detail.    All parts and percentages are expressed by weight
unless otherwise indicated.

Example 1

(1)   Preparation of antibody-producing cells

Normal human stomach tissue membrane components were administered intravenously to new-born C57BL/6 mice within 24 postnatal hours in a dose of 100 µg of proteins per animal.  After the lapse of 8 weeks, the mice were intraperitoneally administered with human stomach cancer tissue membrane components in a dose of 100 µg of proteins per animal together with aluminum hydroxide gel (2 mg per animal) and killed pertussis vaccine ($1 \times 10^9$ per animal), followed by 3-5 immunizations with the same antigen in a dose of 100 µg per animal on the protein basis at one- to two-week intervals.  From among the mice thus immunized, those mice whose antisera reacted intensely with human stomach cancer cells or tissues or membrane fragments were selected, and spleen cells were prepared from such mice and submitted to cell fusion.

(2)   Preparation of mouse myeloma cells

The 8-azaguanine-resistant mouse myeloma cell line P3-U1 was cultivated in normal medium to thereby secure not less than $2 \times 10^7$ cells at the time of cell fusion, and submitted to cell fusion as a parent strain.

(3)   Hybridoma production

The spleen cells and myeloma cells obtained above in (1) and (2), respectively, were used in a ratio of 5:1 (by number) and fusion was carried out by the procedure mentioned hereinabove. After cultivation in HAT medium at 37°C in an atmosphere containing 5% of $CO_2$ for 14 days, fused cells were selected and, after change of the medium to HT medium, cultivation was continued. Based on the results of anti-human stomach cancer antibody titer determination, active wells were selected and, after change of the medium to normal medium, cloning was repeated twice. In this manner the hybridoma strain AMC-382 capable of producing a monoclonal antibody having no reactivity at all with any of normal human cells or tissues or other cancers but having specific reactivity with human stomach cancer, as determined by various assay methods was selected. This hybridoma strain has been deposited with the European Collection of Animal Cell Cultures, Great Britain, as of February 27, 1986 under the deposit number ECACC 86022701 under Budapest Treaty.

(4) Purification of the monoclonal antibody

Pristane-treated 8-week-old female C57BL/6 mice were intraperitoneally injected with the hybridoma strain AMC-382 obtained above in (3) at a dose of 4 x $10^6$ cells per animal. In 10-21 days, the hybridoma produced ascites carcinoma. The ascitic fluid was collected from the ascitic fluid-bearing mice (5-10 ml per animal), deprived of solids by centrifugation (3,000 rpm, 5 minutes), subjected to salting out with 50% ammonium sulfate, dialyzed against PBS supplemented with NaCl (0.5 M), and applied to a Sephacryl (Pharmacia Fine Chemicals) column (bed volume 750 ml) at a flow rate of 15 ml/hr. An IgM fraction was collected and used as a purified antibody.

(5) Specificity characteristics of AMC-382

The reaction specificity characteristics of the thus-obtained anti-stomach cancer-specific monoclonal antibody AMC-382 are shown below in Table 1.

Table 1

|  |  | Antibody AMC-382 |
|---|---|---|
| | Tissues | |
| | Stomach cancer | 7/7 |
| | Intestinal metaplastic stomach | 2/6 |
| | Normal stomach | 0/6 |
| Affinity analysis (ELISA) | Normal tissues other than stomach | 2/12* |
| | CEA | - |
| | Cultured strains | |
| | Stomach cancer | 4/4 |
| | Fetal skin | 0/1 |
| | Cancers other than stomach cancer** | 2/7** |

* Weak reaction with part of large intestine

** Strong reaction with cultured spleen cancer strain;
Large intestine, malignant melanoma, uterus, chorio-
epithelioma, neuroblastoma, leukemia cells, myeloma.

### Example 2

Stomach cancer tissues were immunohistochemically
stained using the monoclonal antibody AMC-382 obtained in
Example 1.  Thus, paraffin-embedded block sections of
stomach cancer tissues excised from 7 stomach cancer
patients were examined.  In all the cases, cancer cells
were stained intensely.  The above results indicated that
pathologic diagnosis of stomach cancer is possible when
AMC-382 is used for immunohistochemical staining.

### Example 3

A preparation of AMC-382 (10 µg/ml) was distributed
in 50-µl portions into the wells of a 96-well plate for
EIA (Flow Laboratories).  After allowing to stand overnight at 4°C, the plate was washed with PBS.  Then, 1%
BSA-PBS was added (200 µl per well).  After overnight
standing, the plate was washed well with PBS.  To the
plate were added 4-fold dilutions of healthy subject-
derived sera (77 samples) or of stomach cancer patient-
derived sera (52 samples) in an amount of 50 µl per
well.  After overnight standing at 4°C, the plate was
washed well with PBS.  Then, a biotin-labeled anti-stomach cancer monoclonal antibody AMC-382 preparation (10 µg AMC-382 per ml) was added as the second antibody in an amount of 100 µl per well. The plate was allowed to stand overnight at 4°C and then washed well with PBS. An avidin-biotin-peroxidase (product of Vector) preparation (10 µg/ml) was distributed in 100-µl portions into the wells. The plate was allowed to stand at room temperature for 1 hour and then washed with PBS. Thereafter, an ABTS substrate solution was added in an amount of 100 µl per well and the reaction was allowed to proceed at room temperature for 30 minutes and then terminated by adding 5% SDS solution (100 µl per well). For each well, the color development was measured using an absorptiometer ($OD_{415}$).

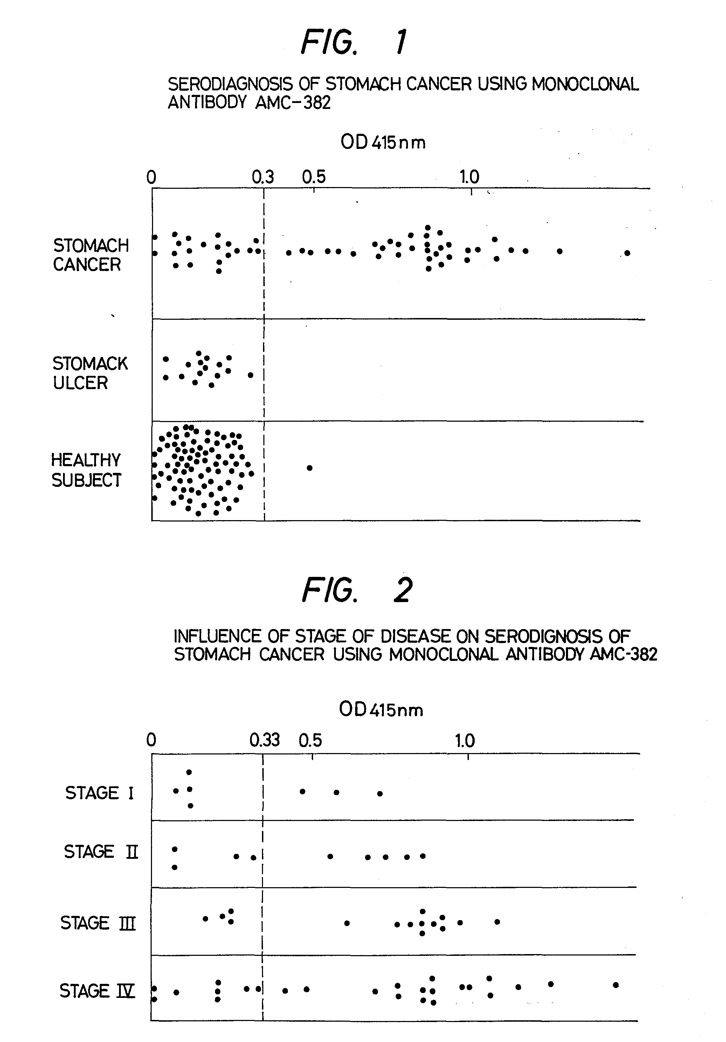

As shown in Fig. 1, the result was positive ($OD_{415} > 0.33$) only for one out of the 77 serum samples from healthy subjects, whereas, for the serum samples from stomach cancer patients, 32 out of the 52 samples gave positive results ($OD_{415} > 0.33$). This indicates that serodiagnosis using the above antibody can detect stomach cancer patients with a probability of 61.5%. Of 16 serum samples from gastric ulcer patients, none gave positive results.

Furthermore, as shown in Fig. 2, where the data obtained for the serum samples from stomach cancer patients are plotted versus the stage of disease, positive results were obtained in 42.8% (3 out of 7 cases) for stage I,

- 21 -

in 55.6% (5 out of 9 cases) for stage II, in 63.6% (7 out of 11 cases) for stage III and in 68% (17 out of 25 cases) for stage IV. When the stages I and II are considered together, the positive rate for early stomach cancer (at stages I and II) amounts to 50% (8 out of 16 cases). This value, when compared with the current detection percentage of 35%, suggests that the above serodiagnostic means might be useful in detecting early stomach cancer. Furthermore, as is evident from Fig. 2, a close correlation can be noted between the blood antigen level and the stage of disease. Therefore, the above serodiagnostic system is also useful in monitoring stomach cancer patients.

### Example 4

For analyzing the antigens which the monoclonal antibody AMC-382 recognizes, stomach cancer tissue membrane components were treated with the enzymes and reagent described below and then examined for the reactivity with AMC-382.

Enzymes and reagent

Trypsin (2.5% solution; Gibco) 0.25% in PBS

Neuraminidase (Boehringer Mannheim) 0.1 U/ml in 0.1 M acetate buffer (pH 4.5)-

3 mM $CaCl_2$

α-L-Fucosidase (Boehringer Mannheim) 0.1 U/ml in 0.1 M phosphate buffer (pH 6.3)

Protease (Sigma) 10 U/ml in 0.1 M phosphate buffer (pH 7.2)

$NaIO_4$ (Wako Pure Chemical Industries) 50 mM in PBS

The stomach cancer tisse membrane components (100 µg proteins per ml) were distributed, in 50-µl portions, into the wells of a plate for EIA (Linbro). After overnight standing at 4°C, the plate was washed with three portions of PBS. Then, 1% BSA-PBS was distributed into the wells (200 µl per well). The plate was allowed to stand at room temperature for 30 minutes to 2 hours and then washed with three portions of PBS. One of the above enzymes or reagent was distributed into the wells (50 µl per well). After 1 hour of reaction at 37°C, the plate was washed with five portions of PBS. Then, the monoclonal antibody AMC-382 (10 µg/ml) was distributed into the wells (50 µl per well), followed by allowing the wells to stand at 4°C for overnight.

After washing with five portions of Tween 20 (Wako Pure Chemical Industries)-PBS, peroxidase-labeled anti-mouse IgG (400-fold dilution) was added in an amount of 50 µl per well, and the reaction was allowed to proceed at room temperature for 2 hours. The plate was washed with five portions of Tween 20-PBS, an ABTS substrate solution was added in an amount of 100 µl and, after 30 minutes of reaction, the absorbance was measured at 415 nm.

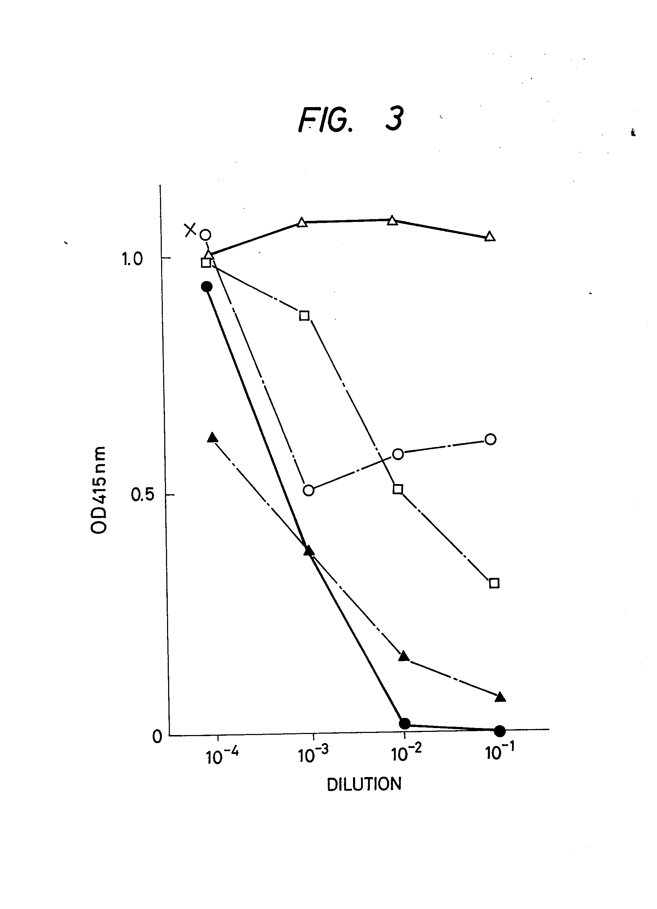

As shown in Fig. 3, the antigenicity disappeared partially upon treatment with periodate (NaIO$_4$) and completely upon treatment with neuraminidase or protease. On the basis of these findings, it was estimated that the monoclonal antibody AMC-382 might recognize sialylated sugar chains.

The present invention thus provide an anti-human stomach cancer monoclonal antibody suited for use in serodiagnosis of stomach cancer.

While the invention has been described in detail and with reference to specific embodiment thereof, it will be apparent to one skilled in the art that various changes and modifications can be made therein without departing from the spirit and scope thereof.

What is Claimed is:

1. An anti-human stomach cancer monoclonal antibody, AMC-382, which belongs to the class IgM and is capable of reacting with human stomach cancer cells, non-reactive with normal human stomach tissues or cells and capable of recognizing the sugar chains of sialylated glycoproteins or glycolipids as antigens.

2. An anti-human stomach cancer monoclonal antibody secreted by a hybridoma having the characteristics of strain ECACC 86022701.

3. A serodiagnosis test composition for detecting the presence of human stomach cancer in a human serum sample, the composition comprising the anti-human stomach cancer monoclonal antibodies AMC-382 of Claim 1, together with a serologically acceptable diluent or carrier.

4. A serodiagnosis test composition for detecting the presence of human stomach cancer in a human serum sample, the composition comprising the anti-human stomach cancer monoclonal antibodies AMC-382 of Claim 2, together with a serologically acceptable diluent or carrier.

5. A serodiagnostic method of detecting the presence of human stomach cancer in a patient comprising:

(a) subjecting a serum sample from the patient to immunoassay with the monoclonal antibody AMC-382 that (1) belongs to the class IgM, (2) is specific to and reacts with human stomach cancer cells, (3) recognizes the sugar chains of sialylated glycoproteins or glycolipids as antigens, and (4) is devoid of reaction with normal human stomach tissue and cells; and

(b) examining the results for the presence of a positive reaction.

6. The serodiagnostic method of Claim 5, in which the anti-human stomach cancer monoclonal antibody is secreted by a hybridoma having the characteristics of strain ECACC 86022701.

7. The diagnostic method of Claim 5, in which the immunoassay method is a sandwich-type enzyme immunoassay.

8. The diagnostic method of Claim 5, in which the immunoassay method is a radioimmunoassay.

9. An immunohistochemical staining method for determining the presence of human stomach cancer cells said method comprising applying to a tissue sample from the stomach of a patient suspected of having stomach cancer a staining amount of the antibodies of Claim 1, and examining the thus-treated tissue sample to determine the selective reaction of the antibodies with stomach cancer cells and the absence of a reaction with the cells of normal human stomach tissue or cells.

10. An immunohistochemical staining method for determining the presence of human stomach cancer cells, said method comprising applying to a tissue sample from the stomach of a patient suspected of having stomach cancer a staining amount of the antibodies of Claim 2, and examining the thus-treated tissue sample to determine the selective reaction of the antibodies with stomach cancer cells and the absence of a reaction with the cells of normal human stomach tissue or cells.

1/2

0 235 817

# FIG. 1

SERODIAGNOSIS OF STOMACH CANCER USING MONOCLONAL
ANTIBODY AMC−382

OD 415 nm

# FIG. 2

INFLUENCE OF STAGE OF DISEASE ON SERODIGNOSIS OF
STOMACH CANCER USING MONOCLONAL ANTIBODY AMC-382

OD 415nm

FIG. 3